(19) Europäisches Patentamt
European Patent Office
Office européen des brevets

(11) Numéro de publication: **0 248 690**
**A1**

(12) **DEMANDE DE BREVET EUROPEEN**

(21) Numéro de dépôt: 87401025.9

(22) Date de dépôt: 05.05.87

(51) Int. Cl.⁴: **C 12 Q 1/70**
C 12 Q 1/68, B 01 L 3/00

(30) Priorité: 06.05.86 FR 8606553

(43) Date de publication de la demande:
09.12.87 Bulletin 87/50

(84) Etats contractants désignés:
AT BE CH DE ES FR GB GR IT LI LU NL SE

(71) Demandeur: **CLONATEC**
**16, rue Dumont d'Urville**
**F-75116 Paris (FR)**

(72) Inventeur: **Lebacq, Philippe**
**33, rue de la Brêche aux Loups**
**F-75012 Paris (FR)**

**Joannes, Martine**
**12, rue du Télégraphe**
**F-75020 Paris (FR)**

**Pouletty, Philippe**
**42, rue Pascal**
**F-75013 Paris (FR)**

(74) Mandataire: **Netter, André et al**
**Cabinet NETTER 40, rue Vignon**
**F-75009 Paris (FR)**

(54) **Procédé et dispositif pour la détection de génomes viraux à ADN et ARN dans les milieux biologiques, notamment dans le sérum sanguin.**

(57) Dispositif pour la détection de génomes viraux à ADN et ARN dans un échantillon de milieu biologique susceptible d'être contaminé par des particules virales.

On traite d'abord l'échantillon pour digérer les acides nucléiques ADN et ARN non viraux et préserver les acides nucléiques viraux. On purifie ensuite, s'il y a lieu, l'échantillon, par exemple en le faisant passer à travers une cartouche (32) contenant un gel pour retenir les protéines présentes dans l'échantillon. Les acides nucléiques viraux sont ensuite extraits et dénaturés en présence d'un mélange d'extraction dans un réservoir (62). Ils sont ensuite recueillis puis détectés par un procédé approprié, par exemple par un procédé colorimétrique.

L'invention permet de réaliser de manière particulièrement simple la détection polyspécifique des génomes viraux à ADN et à ARN, notamment dans des milieux biologiques tels que le sérum.

FIG.3

EP 0 248 690 A1

## Description

### Procédé et dispositif pour la détection de génomes viraux à ADN et ARN dans les milieux biologiques, notamment dans le sérum sanguin.

L'invention concerne la détection des génomes viraux à ADN et ARN dans des échantillons de milieux biologiques susceptibles d'être contaminés par des particules virales.

Elle s'applique en particulier à la détection des virus dans les dérivés sanguins tels que les sérums.

Un des principaux problèmes posés par les transfusions sanguines est celui du risque de contamination virale. Pour prévenir un tel risque, les échantillons sanguins recueillis auprès des donneurs sont systématiquement soumis à des analyses afin de détecter la présence éventuelle de virus. Ils ne sont ensuite utilisés que si les analyses ont donné des résultats négatifs.

Les virus susceptibles de provoquer une telle contamination sont principalement le virus de l'hépatite B et l'agent de l'hépatite non A non B. Aux Etats-Unis, par exemple, on estime à environ 800 000 le nombre des individus porteurs du virus de l'hépatite B. La découverte du Sida est maintenant à prendre en compte et l'on sait que d'autres virus sont transmis par transfusion sanguine. C'est le cas du cytomégalovirus largement répandu dans la population, puisque 70% de la population adulte présente des anticorps contre ce virus.

Or, les tests actuellement utilisés en transfusion sanguine sont monospécifiques et ne permettent de détecter que les virus connus. On se trouve alors dans l'impossibilité de détecter le ou les virus de l'hépatite non A non B puisque ceux-ci ne sont pas encore identifiés à l'heure actuelle.

Par ailleurs, la sensibilité de ces tests connus est insuffisante pour détecter l'ensemble des sérums infectieux.

Parmi les tests utilisés pour dépister un sérum infectieux on distingue :

- les tests de détection des antigènes viraux. C'est le cas, par exemple, des tests de détection du virus de l'hépatite B dont on détecte l'antigène de surface (HBs) par une technique d'agglutination, une technique radio-immunologique ou une technique immuno-enzymatique.

- les tests de détection des anticorps spécifiques. C'est le cas, par exemple, des anticorps anti-LAV ou anti- HTLV III (agents du Sida) et des anticorps anti-cytomégalovirus.

- les tests de détection d'activité enzymatique virale. C'est le cas, par exemple, de la DNA polymérase du virus de l'hépatite B.

- les tests de détection d'activité enzymatique aspécifiques témoins d'une hépatite évolutive. C'est le cas, par exemple, du dosage de transaminases : test peu sensible et peu spécifique qui a été proposé pour l'hépatite non A non B.

- les tests d'hybridation permettant de détecter spécifiquement le génome d'un virus donné. C'est le cas, par exemple, de la détection du génome du virus de l'hépatite B.

Il apparaît, par conséquent, qu'aucun des tests mentionnés ci-dessus ne permet de détecter correctement le ou les virus de l'hépatite non A non B et que chaque test ne permet de détecter qu'un seul type de virus, et cela avec une sensibilité limitée.

La multiplicité des virus à rechercher, le manque de connaissance sur les caractéristiques de certains virus, posent ainsi deux problèmes majeurs pour les transfusions sanguines. Tout d'abord, le nombre des tests à effectuer sur chaque sérum est de plus en plus élevé puisqu'il faut, chaque fois, conduire un test spécifique pour chaque type de virus (virus de l'hépatite B, virus cytomégalovirus, rétrovirus, etc.). En outre, le coût élevé de ces tests est difficilement compatible avec les contraintes techniques et économiques liées aux transfusions sanguines.

Par ailleurs, en dehors des tests mentionnés ci-dessus, il a été proposé d'effectuer une détection des acides nucléiques viraux après leur extraction et leur purification, indépendamment du type de virus recherché.

Cette méthode a été utilisée pour la détection de l'ADN viral dans le sérum de malades atteints d'hépatite B. Cette méthode fait intervenir à la fois une hydrolyse de l'ADN contaminant de l'hôte, une destruction de l'enveloppe et de la capside virale, une extraction de l'ADN viral et son absorption sur un support revêtu de poly-L-lysine suivie d'une détection radio-immunologique à l'aide d'anticorps polyclonaux anti-ADN.

Le but de l'invention est de procurer un procédé pour la détection polyspécifique de génomes viraux à ADN et à ARN dans des milieux biologiques susceptibles d'être contaminés par de tels génomes viraux.

Un autre but de l'invention est de procurer un tel procédé de détection qui soit facilement mis en oeuvre et facilement automatisé.

Un autre but de l'invention est de procurer un tel procédé pouvant être utilisé plus particulièrement pour la détection polyspécifique des génomes viraux à ADN et à ARN dans le sérum sanguin.

Un autre but de l'invention est de procurer un dispositif pour la mise en oeuvre de ce procédé de détection.

L'invention concerne, tout d'abord, un procédé pour la détection de génomes viraux à ADN et ARN dans un échantillon de milieu biologique susceptible d'être contaminé par des particules virales. Ce procédé comprend, pour l'essentiel, les opérations suivantes :

- traitement de l'échantillon de manière à digérer les acides nucléiques ADN et ARN circulants non viraux et à préserver les acides nucléiques viraux;

- extraction des acides nucléiques viraux de l'échantillon traité, par destruction de l'enveloppe et de la capside virale, et dénaturation des acides nucléiques extraits;

- recueil des acides nucléiques viraux extraits et dénaturés; et

- détection des acides nucléiques ainsi recueillis.

Ce procédé est applicable pour la détection des virus de l'hépatite B, de l'hépatite non A non B, du cytomégalovirus, des virus LAV et HTLV III du Sida, et de toute autre particule virale.

Conformément à l'invention, la détection des génomes viraux à ADN et ARN peut être appliquée aux différents milieux biologiques susceptibles de contenir de tels génomes.

Les milieux biologiques concernés sont, principalement ceux d'origine animale, notamment humaine, tels que les dérivés sanguins, en particulier le sérum sanguin, l'urine, le lait, le liquide lacrymal, la salive, le liquide céphalorachidien, le liquide amniotique, les fèces, etc.

On peut citer également les milieux biologiques d'origine végétale, comme la sève des plantes.

En outre, ce procédé peut être appliqué à d'autres milieux biologiques comme les produits alimentaires, tels que les produits laitiers, les eaux, etc.

D'autres exemples de milieux biologiques concernés sont les vaccins, le procédé étant alors utilisé pour vérifier que le vaccin n'est pas contaminé, les surnageants de cultures, etc.

Dans le cas où le procédé est appliqué à un échantillon d'un milieu biologique riche en protéines, comme par exemple un sérum sanguin, le procédé comprend avantageusement une opération intermédiaire qui se déroule après l'opération de traitement par digestion des acides nucléiques circulants non viraux et avant l'opération d'extraction des acides nucléiques viraux. Cette opération intermédiaire consiste à effectuer une purification de l'échantillon traité de manière à retenir les protéines présentes dans l'échantillon.

L'opération de digestion des acides nucléiques contaminants non viraux est effectuée en ajoutant de la DNase et de la RNase, en laissant ensuite réagir le mélange ainsi formé, puis en ajoutant à ce mélange de la RNasine. Grâce à cette opération, on élimine du milieu biologique, en particulier du sérum sanguin, les acides nucléiques contaminants non viraux, par exemple ceux circulant dans le sérum.

Dans le cas où on fait appel à une purification intermédiaire dans le but d'éliminer les protéines de l'échantillon, cette opération comprend avantageusement une filtration sur une cartouche de gel avec élution, sous une différence de pression (dépression ou pression), au moyen d'un tampon d'élution. Il n'est ainsi pas nécessaire de faire appel à des opérations délicates de centrifugation.

L'opération d'extraction et de dénaturation des acides nucléiques viraux s'effectue, avantageusement, par mise en incubation de l'échantillon prétraité avec un mélange d'extraction propre à détruire l'enveloppe et la capside virale et à déproténéiser et dénaturer partiellement les acides nucléiques viraux.

L'opération de recueil des acides nucléiques viraux extraits et dénaturés s'effectue par filtration sur une membrane, par exemple une membrane en polyamide ou en Nylon, telle que du Nylon 66 chargé positivement.

Avantageusement, l'opération de filtration sur cartouche de gel et l'opération de recueil des acides nucléiques viraux s'effectuent successivement en faisant appel à une trompe à vide, ou à tout autre moyen pour accélérer les deux opérations de filtration.

Enfin, l'opération de détection s'effectue avantageusement en utilisant une substance propre à déclencher un signal de détection, cette substance étant conjuguée à une autre substance ayant une affinité pour l'ADN ou l'ARN recueilli.

Dans un mode de réalisation préféré de l'invention, la substance de déclenchement est une enzyme propre à transformer un substrat incolore et soluble en un produit coloré qui précipite in situ.

L'invention vise aussi un dispositif de mise en oeuvre de ce procédé qui comprend essentiellement :

- un support supérieur comprenant au moins un puits propre à recevoir un échantillon de milieu biologique traité;

- un support inférieur adaptable sous le support supérieur et comprenant au moins un réservoir en correspondance du puits du support supérieur, ledit réservoir étant prévu pour recevoir le mélange d'extraction;

- des moyens de communication étanches entre chaque puits et le réservoir correspondant; et

- des moyens pour appliquer une différence de pression entre l'échantillon contenu dans le puits et l'intérieur du réservoir de manière à attirer l'échantillon dans le réservoir.

Selon une autre caractéristique du dispositif, le support supérieur est limité par une face supérieure et une face inférieure, chaque puits ayant une forme générale cylindrique et présentant un fond en entonnoir qui se prolonge par une canule faisant saillie par rapport à la face inférieure du support supérieur.

Chaque puits est propre à recevoir une cartouche ayant la forme générale d'un tube cylindrique dont le fond est muni d'une membrane. Cette cartouche permet ainsi de recevoir un gel propre à retenir les protéines de l'échantillon à analyser.

Selon une autre caractéristique du dispositif de l'invention, le support inférieur est limité par une face supérieure et une face inférieure, et chaque réservoir débouche par son embouchure dans ladite face supérieure, ladite embouchure étant propre à recevoir la canule du puits correspondant du support supérieur lorsque les supports supérieur et inférieur sont adaptés l'un sur l'autre.

Selon une autre caractéristique du dispositif de l'invention, au moins une tubulure est associée à chaque réservoir, ladite tubulure débouchant d'une part à proximité de l'embouchure et, d'autre part, dans la face inférieure, ce qui permet de maintenir une différence de pression entre l'extérieur et l'intérieur du réservoir.

Selon une autre caractéristique de l'invention, le dispositif comprend en outre une embase propre à recevoir le support inférieur et à définir avec celui-ci une chambre susceptible d'être mise en dépression par une source appropriée.

L'invention prévoit également que, dans le but de récupérer les acides nucléiques viraux recueillis dans chaque réservoir, le support inférieur peut être mis en position retournée sur l'embase, avec interposition d'un support intercalaire entre l'embase et le support inférieur en position retournée, et interposition d'une membrane de filtration entre le support inférieur et le support intercalaire. Cette membrane de filtration est destinée à récupérer les acides nucléiques viraux contenus dans les différents réservoirs et cela par filtration sous vide.

Dans la description qui suit, donnée à titre d'exemple, on se réfère aux dessins annexés, sur lesquels :

    - la figure 1 est une vue latérale d'un dispositif de détection conforme à l'invention;

    - la figure 2 est une vue de dessus du dispositif de la figure 1;

    - la figure 3 est une vue en coupe suivant la ligne III-III de la figure 2, le dispositif étant assemblé pour réaliser l'opération de digestion des acides nucléiques non viraux, ainsi que la purification et l'extraction des acides nucléiques viraux; et

    - la figure 4 est une vue en coupe correspondant à celle de la figure 3, dans laquelle le support supérieur du dispositif a été enlevé et le dispositif est assemblé pour réaliser l'opération de recueil des acides nucléiques viraux extraits et dénaturés.

La description détaillée qui suit, donnée uniquement à titre d'exemple, concerne la détection d'acides nucléiques viraux dans des échantillons de sérums, cette détection s'effectuant au moyen du dispositif représenté sur le dessin, lui aussi donné à titre d'exemple.

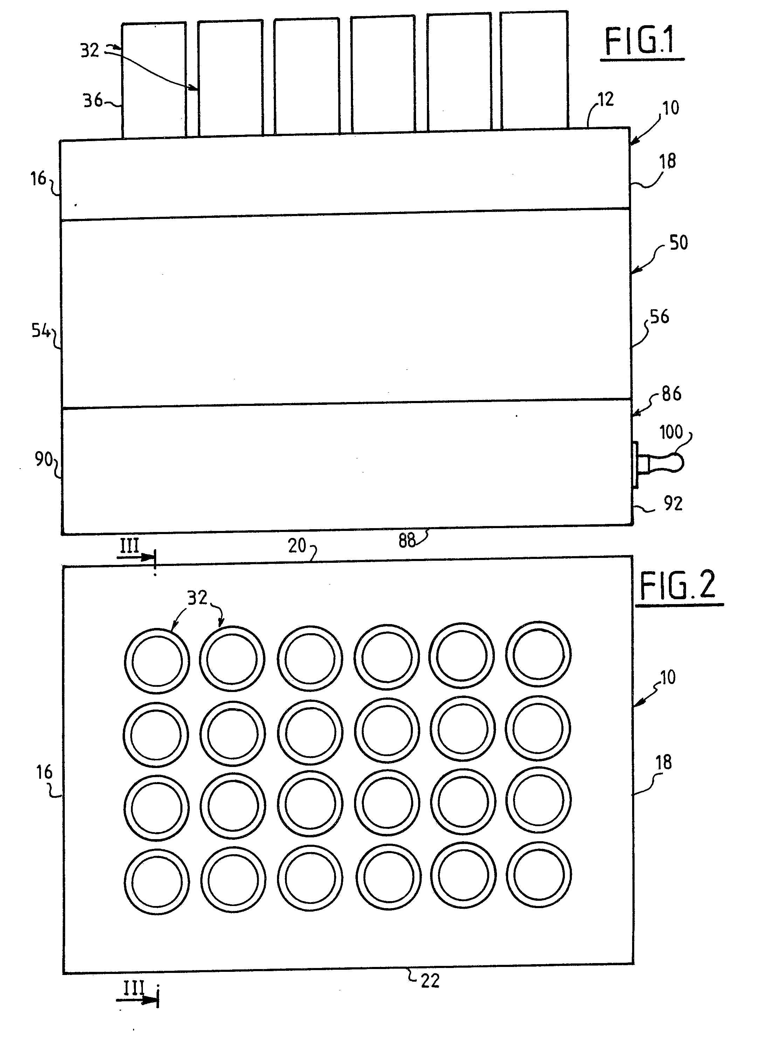

Le dispositif de détection comprend un support supérieur 10 de forme générale parallélépipédique limité par une face supérieure 12, une face inférieure 14, deux faces latérales opposées 16 et 18 et deux autres faces latérales opposées 20 et 22. Dans l'épaisseur du support supérieur 10 sont ménagés des puits 24 disposés à intervalles réguliers. Dans l'exemple, le support ménage quatre rangées de six puits (figure 2).

Chaque puits 24 comprend une partie cylindrique 26 qui, à sa partie supérieure, débouche sur la face supérieure 12 et qui, à sa partie inférieure, se raccorde avec un fond 28 en forme d'entonnoir, lequel se prolonge vers le bas par une canule 30 faisant saillie au-delà de la face inférieure 14.

Chaque puits 24 est destiné à recevoir une cartouche 32 de forme générale cylindrique qui présente une partie inférieure 34 propre à s'emboîter dans le puits 26 correspondant et une partie supérieure 36 de diamètre externe plus grand que celui de la partie 34. La cartouche 32 ménage ainsi un rebord annulaire 38 propre à reposer sur la face supérieure 12 du support 10. La cartouche 32 présente un diamètre intérieur constant et elle est munie, à sa partie inférieure, d'un filtre 40. Chaque cartouche 32 peut ainsi recevoir une certaine quantité d'un gel 42.

Le pourtour de la face inférieure 14 ménage une rainure 44 propre à coopérer par emboîtement avec une nervure correspondante 46 que ménage le pourtour de la face supérieure 48 d'un support inférieur 50. Ce support inférieur est en outre limité par une face inférieure 52, deux faces latérales opposées 54 et 56 et deux autres faces latérales opposées 58 et 60.

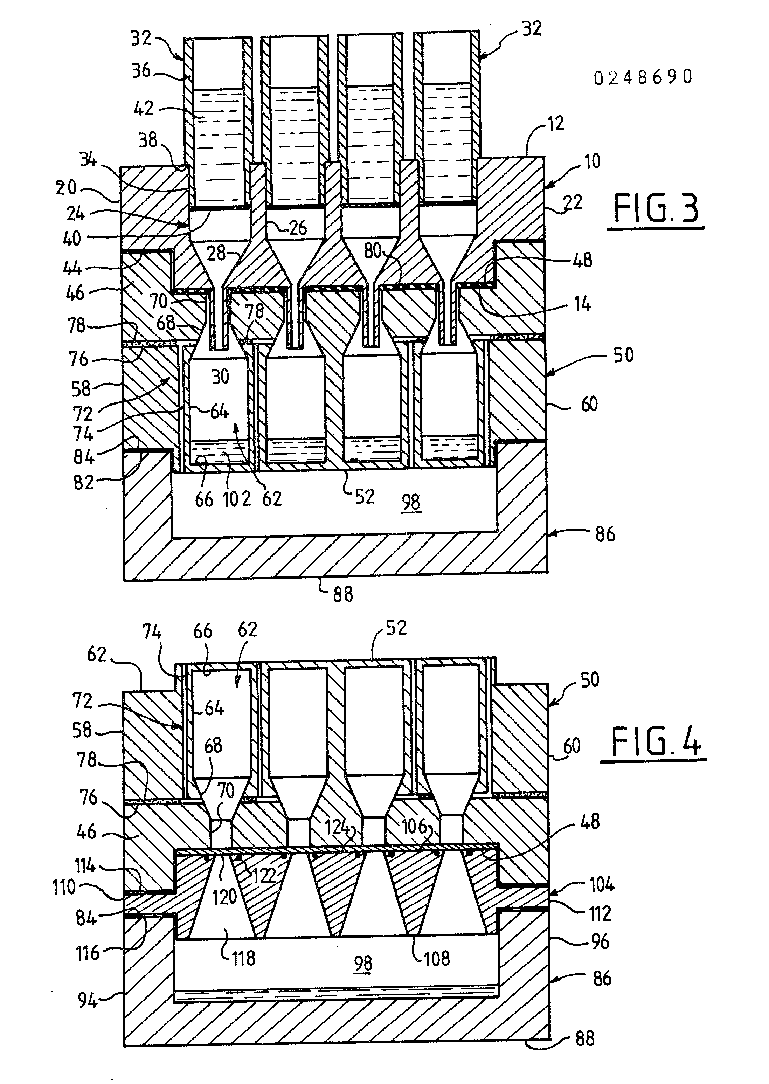

Le support 50 est usiné intérieurement pour ménager vingt-quatre réservoirs 62 correspondant chacun à un puits 24. Chaque réservoir comprend une partie cylindrique 64 raccordée, en partie inférieure, à un fond 66 et, en partie supérieure, à une partie tronconique 68. Cette dernière se raccorde à une embouchure cylindrique 70 qui débouche dans la face supérieure 48. Lorsque les supports 10 et 50 sont assemblés l'un sur l'autre (figure 3), la canule 30 de chaque puits 24 pénètre dans l'embouchure 70 du réservoir 62 correspondant, ce qui permet de récupérer le mélange provenant de la filtration sur le gel 42 de la cartouche correspondante. Pour permettre cette filtration, à chaque réservoir 62 est associée une tubulure 72 qui communique d'une part dans le réservoir 62 à proximité de son embouchure 70 et, d'autre part, dans la face inférieure 52 du support 50. Pour réaliser les tubulures 72, on perce, à partir de la face 52, un certain nombre d'alésages borgnes 74 sur une profondeur telle que l'extrémité de chaque alésage se situe sensiblement au niveau de la partie tronconique 68 des réservoirs 62. On perce ensuite un certain nombre d'alésages horizontaux 76, le perçage s'effectuant à partir des faces latérales 50 et 60. Les alésages 76 sont ensuite bouchés comme montré en 78 de manière que les réservoirs 62 ne communiquent qu'avec les alésages 74.

Pour obtenir l'étanchéité entre les supports 10 et 50, il est avantageusement prévu une feuille 80 en silicone disposée entre la face inférieure 14 du support 10 et la face supérieure 48 du support 50, cette feuille de silicone ménageant vingt-quatre passages pour les canules 30.

La face inférieure 52 du support 50 ménage une rainure périphérique 82 propre à permettre l'emboîtement à joint étanche du support 50 sur le rebord périphérique 84 d'une embase 86. Cette embase 86, de forme générale parallélépipédique, est limitée extérieurement par une face inférieure 88, deux faces verticales opposées 90 et 92 et deux autres faces verticales opposées 94 et 96. L'embase 88 est évidée intérieurement pour ménager une chambre 98 propre à être mise en dépression par l'intermédiaire d'un raccord 100 susceptible d'être relié à une source de dépression.

Dans la position représentée à la figure 3, le dispositif permet d'effectuer simultanément la filtration de vingtquatre échantillons dans les vingt-quatre cartouches 32, le filtrat étant recueilli dans chaque réservoir 62 dans lequel aura été placé au préalable un mélange 102 d'extraction et de dénaturation.

Dans une phase ultérieure du procédé, le support supérieur 10 et les cartouches 32 sont enlevés et le support inférieur 50 est disposé en position retournée sur l'embase 86, avec interposition d'un support intercalaire 104. Ce dernier, de forme générale parallélépipédique, est limité par une face supérieure 106, une face inférieure 108, deux faces latérales opposées 110 et 112 et deux autres faces latérales opposées (non représentées). Les faces 106 et 108 sont réalisées de manière à ménager, sur leur pourtour, respectivement des rainures 114 et 116 propres à coopérer respectivement avec la nervure 46 et avec le rebord 84.

Le support intercalaire 104 ménage vingt-quatre passages de communication de forme tronconique qui s'évasent du haut vers le bas. Dans une autre version, ces passages pourraient être cylindriques. L'ouverture supérieure 120 de chaque passage 118 est en vis-à-vis de l'embouchure 70 du réservoir 62 correspondant en position retournée. Chaque ouverture 120 est entourée par un joint torique d'étanchéité 122. En outre, une membrane de filtration 124 est interposée entre le support intercalaire 104 et le support inférieur 50 en position retournée.

Dans la position représentée à la figure 4, on réalise la filtration sous dépression du contenu de chaque réservoir 62, ce qui permet de retenir sur la membrane 124 les acides nucléiques viraux extraits et dénaturés provenant du réservoir. La tubulure 72 assure alors la mise en communication de l'intérieur du réservoir 62 avec l'atmosphère extérieure, ce qui permet de réaliser la filtration.

Le dispositif tel que décrit précédemment est utilisé de la manière suivante.

On commence par réaliser la digestion des acides nucléiques ADN et ARN contaminants contenus dans les différents échantillons de sérums. Chaque échantillon de sérum, par exemple de 1 millilitre, est traité par l'adjonction de DNase à 50 microgrammes par millilitre en présence d'ions Mg + + et de RNase à 30 microgrammes par millilitre, puis on laisse réagir le mélange ainsi formé pendant environ 30 minutes à la température ambiante. Chaque sérum est ensuite traité par de la RNasine de manière à avoir la certitude que toute la RNase a été détruite.

Chaque échantillon de sérum ainsi traité est ensuite introduit dans une cartouche 32 remplie d'un gel 42 destiné à retenir les protéines présentes dans l'échantillon.

Le gel de filtration utilisé peut être par exemple celui commercialisé sous la dénomination Séphacryl S300 par la firme Pharmacia. Ce gel permet de réaliser une filtration rapide et une séparation grossière du fait que la particule virale représente une masse moléculaire très élevée, à savoir plusieurs dizaines ou plusieurs centaines de millions de Dalton, et doit si possible être éluée dans le volume mort du gel. Le gel Séphacryl S300 est un mélange de bisacrylamide et de dextran qui constitue un réseau poreux assez rigide dans lequel les molécules s'engagent d'autant mieux que leur masse moléculaire est petite. Les molécules de petite masse moléculaire, retardées par un chemin plus long à parcourir, sont éluées en dernier. Ce gel permet la séparation des molécules de masse moléculaire comprise entre 10 000 et 1 500 000 Dalton selon un Kav compris, respectivement, entre 0,8 et 0,1.

On applique sur chaque cartouche un millilitre de sérum que l'on élue par aspiration, la chambre 98 de l'embase 86 étant mise sous dépression. L'élution s'effectue avec un millilitre d'un tampon Tris 50 mM, 25 mM NaCl, 10 mM MgCl₂, à pH 8,0. Le filtre 40 destiné à retenir le gel 42 dans chaque cartouche est avantageusement constitué par une membrane en polyamide, comme par exemple la membrane Blutex commercialisée par la firme Tripette et Renaud, une telle membrane ayant une ouverture de maille de 125 micromètres.

A la sortie de chaque cartouche, l'éluat s'écoule sur le fond 28 du puits correspondant et tombe dans le réservoir 62 correspondant, en passant par la canule 30.

Dans chaque réservoir a été introduit au préalable un mélange propre à permettre l'extraction des acides nucléiques viraux contenus dans l'éluat ainsi recueilli. Il s'agit d'un mélange d'extraction dénaturant destiné à extraire les acides nucléiques protégés par les enveloppes et les capsides des virus, en faisant appel à un agent dénaturant tel que le formamide.

On utilise avantageusement un mélange composé de formamide à 70%, 100 mM EDTA, NaClO₄ 2M. Le formamide a essentiellement pour effet de détruire les liaisons hydrogène à la fois des protéines et de l'ADN et ainsi de dénaturer les protéines et l'ADN. L'EDTA sert à piéger les ions divalents impliqués dans les structures protéiques et nucléiques. Il sert également à piéger le magnésium cofacteur des nucléases. On est ainsi assuré que les traces de DNase, s'il en subsistait, seraient dénaturées et inactives.

Le mélange ainsi contenu dans chaque réservoir 62 est mis en incubation à 37°C pendant environ 3 heures. Pour cela, il suffit simplement de placer le dispositif, ou plus simplement le support inférieur 50 seul, à l'intérieur d'un incubateur, après agitation manuelle pendant une minute.

L'incubation étant terminée, on retourne le support inférieur 50 et on le dispose sur le support intercalaire 104 avec inter position d'une membrane 124, comme représenté à la figure 4. Cette membrane est choisie de manière à permettre le recueil des acides nucléiques dénaturés. Différents types de membranes peuvent être utilisés dans ce but, par exemple des membranes en polyamide ou nylon, en nitrocellulose, en DEAE-cellulose, en acétate de cellulose, en fibres de verre, en holofibres, etc.

Dans un mode de réalisation préféré de l'invention, on utilise une membrane commercialisée sous la dénomination Gene Screen + par la firme New England Nuclear. Il s'agit d'une membrane en Nylon 66 chargé, ayant des pores d'ouverture comprise entre 0,22 et 0,45 micromètres. Un autre exemple de membrane est celle commercialisée sous la dénomination Nytran par la Firme Schleicher et Schuell. De telles membranes permettent d'obtenir une fixation d'acides nucléiques viraux triple de celle des membranes en nitrocellulose, en raison des charges positives de la membrane. En outre, il s'agit de membranes solides, d'utilisation facile puisqu'il n'est pas nécessaire de réaliser une cuisson sous vide, et ne faisant appel qu'à des tampons simples. En outre, ces membranes ont l'avantage de permettre une révélation colorimétrique aisée des acides nucléiques ainsi recueillis.

La filtration sur la membrane 124 s'effectue sous vide peu élevé et pendant une durée d'environ 5 minutes.

On enlève ensuite le support 50 pour récupérer la membrane de filtration 124. Cette membrane est ensuite séchée avant d'être soumise à une analyse afin de révéler la présence éventuelle d'acides nucléiques viraux recueillis sur la membrane.

La révélation des acides nucléiques viraux peut se faire par différents procédés, en particulier des procédés comprenant l'utilisation d'une substance propre à déclencher un signal de détection et conjuguée à une substance ayant une affinité pour l'ADN ou l'ARN.

La substance propre à déclencher le signal de détection peut être une enzyme, par exemple la phosphatase alcaline, la peroxydase, la bêta-galactosidase, la glucose-oxydase, etc, un isotope tel que $I^{125}$, un fluorochrome, l'or colloïdal, etc.

La substance ayant une affinité pour l'ADN ou l'ARN peut être, par exemple, la protamine, la poly-L-lysine, la spermine, la spermidine, des anticorps anti-ADN et anti-ARN (polyclonaux ou monoclonaux), les protéines histones, des ions métalliques, etc.

Dans un mode de réalisation préféré de l'invention, la substance de déclenchement est une enzyme propre à transformer un substrat incolore et soluble en un produit coloré qui précipite in situ.

Dans ce mode de réalisation préféré de l'invention, la membrane préalablement séchée est ensuite saturée pendant environ 30 minutes avec une solution propre à bloquer les sites non spécifiques de la membrane, c'est-à-dire les sites de la membrane qui ne sont pas déjà occupés par des acides nucléiques viraux. Cette opération de saturation peut s'effectuer par immersion de la membrane dans la solution de saturation ou encore par filtration de cette solution de saturation à travers la membrane.

A titre d'exemple, cette solution de saturation peut être une solution de protéines de lait à 370 milligrammes par millilitre, de BSA (sérum albumine bovine) à 37 milligrammes par millilitre, de Tween à 0,3% dans un tampon 50 mM Tris-HCl pH8, EDTA 1 mM, NaCl 25 mM (tampon TEN). La membrane est ensuite mise au contact d'une solution de protamine ou de poly-L-lysine couplée à des molécules de phosphatase alcaline par l'intermédiaire d'une liaison peptidique.

Cette incubation s'effectue pendant environ 15 minutes à la température ambiante. La protamine-phosphatase alcaline est diluée dans la solution bloquante décrite précédemment. La membrane est ensuite lavée par filtration avec une solution de Brig à 0,3%, 0,5 M NaCl (20 millilitres par centimètre carré).

Dans l'exemple, la détection est réalisée par l'utilisation d'un système colorimétrique. Le procédé colorimétrique, en lui-même connu, utilise la transformation d'un naphtolphosphate en naphtol par la phosphatase alcaline. Le naphtol peut alors réduire un produit, tel que celui connu sous la dénomination Fast-Red, et ainsi transformer un substrat incolore et soluble en un produit coloré et insoluble qui précipite in situ.

Un autre exemple de système colorimétrique est celui utililisant le BCIP (5-bromo-4-chloro-3-indolyl-phosphate) et le NBT (nitro-bleu de tétrazolium).

La lecture s'effectue de visu ou bien par l'intermédiaire d'un réflectomètre. Dans le cas où la membrane 124 a effectivement recueilli des acides nucléiques viraux, on détecte, à l'emplacement de la filtration, une tache colorée révélant l'existence des acides nucléïques recherchés.

Bien entendu, la révélation des acides nucléiques recueillis peut se faire par d'autres procédés, notamment par des tests de détection d'ADN par hybridation. Ainsi, dans le cas du virus de l'hépatite B, l'ADN peut être détecté par une sonde. Il s'agit là cependant d'un test spécifique du virus à rechercher.

L'invention est susceptible de nombreuses variantes de réalisation. Par exemple, la purification de l'échantillon préalablement traité peut se faire par mélange avec un gel magnétique avec élution à l'aide d'un aimant permanent. Le gel magnétique est, par exemple, celui commercialisé sous la dénomination Magnogel par la Firme IBF.

Le modèle expérimental retenu pour la mise au point du procé dé et du dispositif de l'invention est le phage lambda. Celui-ci présente en effet de nombreux avantages : il est facilement cultivable et titrable; on peut en récolter de grandes quantités et en introduire des quantités déterminées dans le sérum; sa taille est de l'ordre de 50 nm et se situe presque à la limite inférieure des plus petits virus (environ une dizaine de nm); et il n'est pas pathogène pour l'homme.

Le rendement du dispositif de filtration et d'extraction des virus, ainsi mis au point avec le phage-lambda, est de l'ordre de 60%. La sensibilité obtenue avec une sonde marquée chimiquement, puis hybridée avec l'ADN retenu sur le filtre, permet la détection d'environ 1 picogramme d'ADN.

La détection directe de l'ADN du phage-lambda à l'aide du système protamine liée de façon covalente à la phosphatase alcaline est de l'ordre de la dizaine de femtogrammes d'ADN détecté. Ce système permet une détection effective jusqu'à 5 à 10 femtogrammes d'ADN ou d'ARN.

Le procédé de l'invention est polyspécifique et, par ailleurs, la présence d'ADN ou d'ARN libre dans le sérum, jusqu'à des concentrations de 10 microgrammes par millilitre, n'introduit pas de faux positifs détectables par le procédé.

Dans des sérums positifs en antigène HBs et en ADN viral par la technique d'hybridation, le procédé de l'invention a donné un résultat positif.

Les sérums HBs et ADN viral négatifs testés parallèlement ont été négatifs par le procédé de l'invention.

Le procédé de l'invention est beaucoup plus sensible que les procédés de tests monospécifiques connus. Le seuil de sensibilité du procédé de l'invention est de $10^3$ à $10^4$ particules virales par millilitre, alors que la sensibilité des tests connus est de $10^6$ à $10^8$ particules virales par millilitre.

**Revendications**

1. Procédé pour la détection de génomes viraux à ADN et ARN dans un échantillon de milieu biologique susceptible d'être contaminé par des particules virales, caractérisé par les opérations suivantes :

- traitement de l'échantillon de manière à digérer les acides nucléiques ADN et ARN circulants non viraux et à préserver les acides nucléiques viraux des particules virales;

- extraction des acides nucléiques viraux de l'échantillon traité, par destruction de l'enveloppe et de la capside virale, et dénaturation des acides nucléiques extraits;

- recueil des acides nucléiques viraux extraits et dénaturés; et

- détection des acides nucléiques viraux ainsi recueillis.

2. Procédé selon la revendication 1, caractérisé par l'opération intermédiaire comprenant, après l'opération de traitement par digestion, la purification de l'échantillon traité de manière à retenir les protéines présentes dans l'échantillon.

3. Procédé selon l'une des revendications 1 et 2, caractérisé en ce que la digestion des acides nucléiques contaminants non viraux est effectuée en ajoutant à l'échantillon de la DNase et de la RNase, en laissant ensuite réagir le mélange ainsi formé, puis en ajoutant à ce mélange de la RNasine.

4. Procédé selon la revendication 3, caractérisé en ce que la digestion est réalisée par adjonction de DNase à 50 microgrammes par millilitre en présence d'ions Mg + + et de RNase à 30 microgrammes par millilitre, pendant environ 30 minutes à la température ambiante.

5. Procédé selon l'une des revendications 2 à 4, caractérisé en ce que la purification de l'échantillon préalablement traité comprend la filtration sur une cartouche de gel avec élution, sous une différence de pression, au moyen d'un milieu tampon.

6. Procédé selon la revendication 5, caractérisé en ce que le gel comprend un mélange de bisacrylamide et de dextran et le tampon d'élution comprend un mélange Tris, NaCl et $MgCl_2$ à pH 8,0.

7. Procédé selon l'une des revendications 2 à 4, caractérisé en ce que la purification de l'échantillon préalablement traité comprend le mélange avec un gel magnétique avec élution à l'aide d'un aimant permanent.

8. Procédé selon l'une des revendications 1 à 7, caractérisé en ce que l'extraction et la dénaturation des acides nucléiques viraux s'effectue par mise en incubation avec un mélange d'extraction propre à détruire l'enveloppe et la capside virale et à déprotéiniser partiellement et dénaturer les acides nucléiques viraux.

9. Procédé selon la revendication 8, caractérisé en ce que le mélange d'extraction comprend du formamide à 70%, 100 mM EDTA et $NaClO_4$ 2M, et que l'incubation est maintenue pendant environ 3 heures à 37°C.

10. Procédé selon l'une des revendications 1 à 9, caractérisé en ce que le recueil des acides nucléiques viraux est obtenu par filtration sur une membrane, par exemple en polyamide.

11. Procédé selon la revendication 10, caractérisé en ce que la membrane est ensuite séchée puis saturée par une solution propre à bloquer les sites de la membrane non occupés par les acides nucléiques viraux.

12. Procédé selon l'une des revendications 1 à 11, caractérisé en ce que l'opération de détection comprend l'utilisation d'une substance propre à déclencher un signal de détection et conjuguée à une substance ayant une affinité pour l'ADN ou l'ARN.

13. Procédé selon la revendication 12, caractérisé en ce que la substance propre à déclencher un signal de détection est choisie parmi des enzymes, par exemple la phosphatase alcaline, des isotopes, par exemple $I^{125}$, les fluorochromes, l'or colloïdal, etc.

14. Procédé selon l'une des revendications 12 et 13, caractérisé en ce que la substance ayant une affinité pour l'ADN ou l'ARN est choisie parmi la protamine, la poly-L-lysine, la spermine, la spermidine, les anticorps anti-DNA et anti-RNA, les histones, les ions métalliques, etc.

15. Procédé selon l'une des revendications 12 à 14, caractérisé en ce que l'opération de détection comprend l'utilisation de protamine couplée à de la phosphatase alcaline.

16. Procédé selon l'une des revendications 12 à 15, caractérisé en ce que la substance déclenchant le signal de détection est une enzyme propre à transformer un substrat incolore et soluble en un produit coloré qui précipite in situ.

17. Procédé selon l'une des revendications 1 à 16, caractérisé en ce que l'échantillon de milieu biologique est choisi parmi les fluides biologiques humains ou animaux, en particulier le sérum, les fluides biologiques d'origine végétale, par exemple la sève des plantes, les produits alimentaires, les vaccins, etc.

18. Dispositif pour la mise en oeuvre du procédé selon l'une des revendications 1 à 17, caractérisé en ce qu'il comprend :

- un support supérieur (10) comprenant au moins un puits (24) propre à recevoir un échantillon de milieu biologique;

- un support inférieur (50) adaptable sous le support supérieur (10) et comprenant au moins un réservoir (62) en correspondance du puits (24) du support supérieur (10), ledit réservoir (62) étant prévu pour recevoir un mélange d'extraction (102);

- des moyens (30, 80) de communication étanche entre chaque puits (24) et le réservoir (62) correspondant; et

- des moyens (96, 98) pour appliquer une différence de pression entre l'échantillon contenu dans le puits (24) et l'intérieur du réservoir (62) de manière à attirer l'échantillon dans le réservoir.

19. Dispositif selon la revendication 18, caractérisé en ce que le support supérieur (10) est limité par une face supérieure (12) et une face inférieure (14) et en ce que chaque puits (62) est de forme générale cylindrique et présente un fond (28) en entonnoir qui se prolonge par une canule (30) faisant saillie par rapport à la face inférieure (14) du support supérieur (10).

20. Dispositif selon la revendication 19, caractérisé en ce que chaque puits (24) est propre à recevoir une cartouche (32) ayant la forme générale d'un tube cylindrique dont le fond est muni d'un filtre (40), ladite cartouche étant propre à recevoir une substance (42), par exemple un gel, pour retenir les protéines de l'échantillon.

21. Dispositif selon la revendication 19, caractérisé en ce que le support inférieur (50) est limité par une face supérieure (48) et une face inférieure (52) et que chaque réservoir (62) débouche par son embouchure (70) dans ladite face supérieure (48), ladite embouchure étant propre à recevoir la canule (30) du puits (24) correspondant du support supérieur (10) lorsque les supports supérieur (10) et inférieur (50) sont adaptés l'un sur l'autre.

22. Dispositif selon la revendication 21, caractérisé en ce qu'au moins une tubulure (72) est associée à chaque réservoir (62), ladite tubulure débouchant, d'une part, à proximité de l'embouchure (70) du réservoir et, d'autre part, dans la face inférieure (52).

23. Dispositif selon la revendication 21, caractérisé en ce qu'il comprend en outre une embase (86) propre à recevoir à joint étanche le support inférieur (50) et à définir avec celui-ci une chambre (98) susceptible d'être mise en dépression par une source appropriée.

24. Dispositif selon la revendication 23, caractérisé en ce que, dans le but de récupérer les acides nucléiques viraux recueillis dans chaque réservoir (62), le support inférieur (50) peut être mis en position retournée sur l'embase (86), avec interposition d'un support intercalaire (104) entre l'embase (86) et le support inférieur en position retournée, et interposition d'une membrane de filtration (124) entre le support inférieur (50) et le support intercalaire (104).

25. Dispositif selon la revendication 24, caractérisé en ce que le support intercalaire (104) est limité par une face supérieure (106) et une face inférieure (108) et qu'il définit une pluralité de passages de communication (118) de forme tronconique ou cylindrique qui s'évasent du haut vers le bas, l'ouverture supérieure (120) d'un passage (118) étant en vis-à-vis de l'embouchure (70) du réservoir (62) correspondant.

0248690

FIG.1

FIG.2

0 2 4 8 6 9 0

FIG.3

FIG.4

Office européen
des brevets

**RAPPORT DE RECHERCHE EUROPEENNE**

Numéro de la demande

EP 87 40 1025

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 427 415 (P.H. CLEVELAND) * En entier * | 18 | C 12 Q 1/70 C 12 Q 1/68 B 01 L 3/00 |
| A | | 19-25 | |
| | --- | | |
| X | FR-A-2 091 793 (WILSON PHARMACEUTICAL & CHEMICAL CORP.) * Page 1, ligne 1 - page 5, ligne 3; page 6, ligne 26 - page 8, ligne 16; figures * | 18 | |
| A | | 19-25 | |
| | --- | | |
| X | EP-A-0 118 735 (MILLIPORE CORP.) * En entier * | 18 | |
| | --- | | **DOMAINES TECHNIQUES RECHERCHES (Int. Cl.4)** |
| A | EP-A-0 145 356 (NATIONAL RESEARCH DEVELOPMENT CORP.) * Abrégé; page 1, ligne 1 - page 2, ligne 26; page 7, ligne 15 - page 8, ligne 10; page 9, lignes 10-22 * | 1,2,17 | C 12 Q G 01 N B 01 L C 12 M |
| | --- | | |
| A | EP-A-0 135 159 (CETUS CORP.) * Abrégé; page 1, ligne 24 - page 4, ligne 19 * | 1,13, 14,17 | |
| | ---    -/- | | |

Le présent rapport de recherche a été établi pour toutes les revendications

| Lieu de la recherche | Date d'achèvement de la recherche | Examinateur |
|---|---|---|
| LA HAYE | 17-09-1987 | HITCHEN C.E. |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 165 633 (JANSSEN PHARMACEUTICA N.V.) <br> * Page 2, ligne 31 - page 3, ligne 30 * | 13 | |
| A,P | JOURNAL OF IMMUNOLOGICAL METHODS, vol. 89, no. 1, ma 1986, pages 123-130, Elsevier Science Publishers B.V. (Biomedical Division), Amsterdam, NL; S.J. BOGUSLAWSKI et al.: "Characterization of monoclonal antibody to DNA - RNA and its application to immunodetection of hybrids" <br> * Page 123 * | 14 | |
| A | CHEMICAL ABSTRACTS, vol. 102, no. 21, 27 mai 1985, pages 296-297, résumé no. 181796g, Columbus, Ohio, US; J.L. ROTI et al.: "DNase I sensitivity of nuclear DNA measured by flow cytometry", & CYTOMETRY 1985, 6(2), 101-8 | 3,4,13 ,14 | DOMAINES TECHNIQUES RECHERCHES (Int. Cl.4) |
| | ---      -/- | | |

Le présent rapport de recherche a été établi pour toutes les revendications

| Lieu de la recherche | Date d'achèvement de la recherche | Examinateur |
|---|---|---|
| LA HAYE | 17-09-1987 | HITCHEN C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82

Office européen
des brevets

**RAPPORT DE RECHERCHE EUROPEENNE**

Numéro de la demande

EP 87 40 1025

Page 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 91, no. 17, 22 novembre 1979, pages 480-481, résumé no. 138668j, Columbus, Ohio, US; S. AOTSUKA et al.: "Measurement of anti-double-stranded DNA antibodies in major immunoglobulin classes", & J. IMMUNOL. METHODS 1979, 28(1-2), 149-62 ----- | 14 | |

DOMAINES TECHNIQUES
RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 17-09-1987 | HITCHEN C.E. |